# EUROPÄISCHE PATENTSCHRIFT

(11) **EP 2 349 426 B1**
(45) Veröffentlichungstag und Bekanntmachung des Hinweises auf die Patenterteilung: **10.09.2014**
(21) Anmeldenummer: 09759867.6
(22) Anmeldetag: 19.11.2009
(51) Int. Cl.: A61M 16/04

(54) **LARYNXMASKE MIT EINEM STUTZEN**
LARYNX MASK HAVING A CONNECTOR
MASQUE LARYNGÉ POURVU D'UNE TUBULURE

(30) Priorität: 27.11.2008 CH 18602008
(43) Veröffentlichungstag der Anmeldung: 03.08.2011
(73) Patentinhaber: Singularity AG, 8124 Maur (CH)
(72) Erfinder: DUBACH, Werner, F., 8124 Maur (CH)
(74) Vertreter: Schneider Feldmann AG Patent- und Markenanwälte
(86) Internationale Anmeldenummer: PCT/CH2009/000370
(87) Internationale Veröffentlichungsnummer: WO 2010/060224

(56) Entgegenhaltungen:
- EP-A- 1 938 855
- WO-A-2006/125986
- US-A- 5 878 745
- US-A1- 2003 037 790
- US-A1- 2005 229 933
- US-B2- 7 040 322

## Beschreibung

Die vorliegende Erfindung betrifft eine Larynxmaske gemäss Oberbegriff des Patentanspruchs 1. Larynxmasken dieser Bauart werden mittels eines Tubus, eines sogenannten supraglottischen Tubus, durch den mittleren Rachen über den Kehlkopfdeckel eines anästhesierten Patienten eingeführt. Larynxmasken werden meist als Einheit zusammen mit den daran angeformten oder befestigten supraglottischen Tubus angeboten. Dieser dient der Offenhaltung der Atemwege und zur Beatmung eines Patienten. Gleichzeitig erlauben solche Larynxmasken auch das Einführen von Tuben, Sonden, optischen Instrumenten und anderen Instrumenten in die Atemwege.

Immer häufiger verfügen solche Larynxmasken über einen Oesophagealzugang. Dieser erlaubt das Einführen von Sonden in die Speiseröhre und in den Magen, um Magensaft und andere Flüssigkeiten sowie Luft aus dem Magen abzusaugen. Das Entleeren des Magens bei anästhesierten Patienten soll verhindern, dass Mageninhalt in die oberen Atemwege zurückfliesst und in die ungeschützten Atemwege (Luftröhre, Bronchien und Lungen) aspiriert wird. Ein weiterer Vorteil eines oesophagealen Zugangs ist das Entweichen von passiv oder aktiv regurgitiertem Mageninhalt aus dem oberen Oesophagus nach aussen und stellt somit einen begrenzten, und damit ungenügenden Aspirationsschutz dar.

Auf dem Markt ist eine grosse Anzahl unterschiedlicher Larynxmasken bekannt. Ein typisches Beispiel zeigt die US 5878745. Hier ist eine Gastro-Laryngealmaske gezeigt bei der der supraglottische Tubus ein Rohr ist, durch das mehrere Schläuche geführt werden. Diese Schläuche haben Lumen, die für die Beatmung und für einen oesophagealen Zugang dienen. Hierbei muss der oesophageale Zugang als Schlauch durch die gesamte Larynxmaske hindurch geführt, verklebt und durch einen Ausgang, welcher den Cuff durchsetzt geführt werden. Dies ist ausgesprochen aufwendig und bedingt viel Handarbeit.

Das Einführen einer Larynxmaske ist nicht immer einfach. Larynxmasken mit einem relativ starren supraglottischen Tubus lassen sich einfacher einführen, wobei die Steifigkeit eine Anpassung der Position der Larynxmaske an die anatomischen Gegebenheiten verhindert. Die Einführung mittels relativ starren supraglottischen Tuben in den Rachenraum können zu Verletzungen führen und die Positionierung im Rachenraum ist nicht immer zuverlässig.

Hochflexible Larynxmasken mit entsprechend supraglottischen hochflexiblen Tuben erlauben eine bessere Positionierung um den Kehlkopf, sind aber schwieriger und damit gelegentlich traumatisch zum Einführen und schwieriger in der Positionierung im Rachenraum. Insbesondere kommt es immer wieder vor, dass bei solchen hochflexiblen Larynxmasken bei der Einführung das proximale Ende der Larynxmaske die sogenannte Spitze, umgeknickt wird. Damit ist eine zuverlässige Dichtung der Larynxmaske nicht mehr gegeben. Um dieses Problem zu beheben, kann man auf ein biegesteiferes Material ausweichen, wobei dann allerdings die Vorteile der hochflexiblen Materialien verloren gehen. Traumatische Effekte im mittleren Rachenraum sind entsprechend die Folge. Auch mit einem leicht erhöhten Luftdruck im Cuff lässt sich das Problem nicht immer zuverlässig lösen. Auch wird bei den heute bekannten Larynxmasken der Oesophagealdurchgang immer durch den Cuff hindurch geführt. Dies kompliziert die gesamte Herstellung der Larynxmaske. Tritt eine Knickung oder auch nur eine etwas stärkere Biegung der Spitze der Larynxmaske auf, so ist damit der Oesophagealdurchgang meist schon nicht mehr frei und ein Instrumentarium oder eine Sonde lässt sich gar nicht oder kaum mehr durchführen.

Bereits aus der EP-1938855 ist eine Larynxmaske bekannt mit einemgeradlinigen Verlauf des Oesophageallumens bekannt. Diese Lösung besitzt jedoch keinen aufblasbaren Cuff und das Oesophageallumen ist durch den Cuff hindurch geführt.

Bereits aus der US 7040322 ist eine Larynxmaske bekannt, bei der die Larynxmaske selber durch die Durchführung eines Oesophagealen Zugangs in Längsrichtung versteift wird. Dieser Oesophageale Zugang wird hierbei in das Zentrum verlegt. Hierdurch wird zwar die Längssteifigkeit der Larynxmaske erhöht, doch wird hierdurch diese in der Bauhöhe ungünstig beeinflusst. Zudem muss mittels einem zusätzlichen aufblasbaren dorsalen Cuff der Rachen des Patienten geschützt werden.

Aus der US 2003/0037790 sind unterschiedliche Varianten von Larynxmasken bekannt, bei der der oesophageale Durchgang entweder durch einen Schlauch durch den Cuff oder über diesen jeweils in Kurven geführt ist.

Eine aufwendig herstellbare Larynxmaske zeigt die US 7040322 bei der der Einführungstubus von ausserhalb des Patienten durchgehend bis zur Spitze des oesophagealen Ausgangs verläuft und zwei integral angeformte Cuffs einmal zur Begrenzung des Respirationsraumes und andererseits zur Dichtung des oesophagealen Ausgangs vorhanden sind.

Letztlich ist aus der WO 2006/125986 eine Larynxmaske gemäss Oberbegriff des Patentanspruchs 1 bekannt, bei dem der Oesophageallumen integral in der Larynxmaske gebildet ist und den Cuff durchsetzt.

Es ist daher die doppelte Aufgabe der vorliegenden Erfindung neben der Meidung der beschriebenen Nachteile des Standes der Technik eine Larynxmaske derart zu verbessern, dass diese einfacher in der Fertigung ist und dass trotzdem auch bei Verwendung eines hochflexiblen Materiales, die genannten Probleme nicht mehr auftreten, respektive deren Auftreten wesentlich reduziert werden.

In den Zeichnungen ist ein bevorzugtes Ausführungsbeispiel des Erfindungsgegenstandes dargestellt, und anhand der nachfolgenden Beschreibung erläutert.

Es zeigt:
- Fig. 1: eine perspektivische Gesamtansicht einer erfindungsgemässen Larynxmaske mit Blick auf die dorsale Deckplatte, während
- Fig. 2: dieselbe Larynxmaske wiederum in perspektivischer Gesamtansicht zeigt jedoch mit Blick auf den ventral angeordneten Cuff.
- Fig. 3: zeigt eine vertikale Aufsicht auf die erfindungsgemässe Larynxmaske auf der ventralen Seite und
- Fig. 4: zeigt einen Längsschnitt durch die Larynxmaske in der Verlaufsrichtung des Oesophagealdurchganges.
- Fig. 5: zeigt einen Querschnitt durch die Larynxmaske im Bereich einer Einführungsstrecke in Blickrichtung zum proximalen Ende und des Oesophagealausganges 6.
- Fig. 6: zeigt wiederum einen Querschnitt durch dieselbe Larynxmaske etwa im mittigen Bereich der Längserstreckung der Larynxmaske wobei die Blickrichtung wiederum zum proximalen Ende gerichtet ist, während
- Fig. 7: denselben Querschnitt an derselben Stelle zeigt jedoch mit Blickrichtung zum distalen Ende der Larynxmaske.

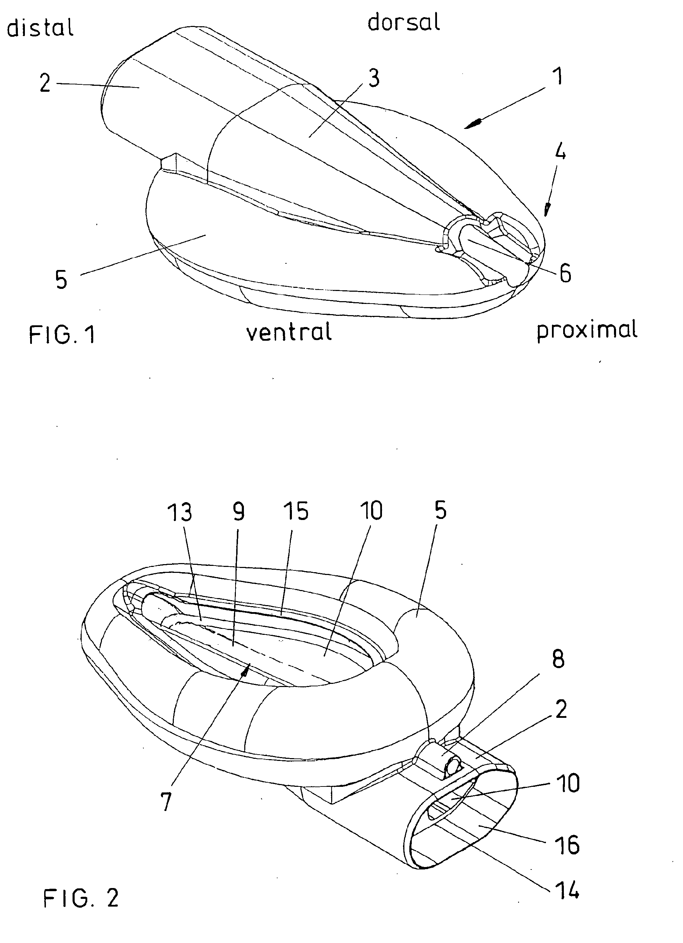

In der Figur 1 erkennt man die Larynxmaske, die insgesamt mit 1 bezeichnet ist. An dieser Larynxmaske wird ein Einführungstubus angeschlossen, der jedoch in der Zeichnung nicht dargstellt ist. Hierzu dient jedoch ein mit 2 bezeichneter Tubusverbindungsstutzen. Dieser Tubusverbindungsstutzen 2 geht über in eine Deckplatte 3 und einem ebenfalls einstückig angeformten Cuff 5. Die gesamte Larynxmaske 1 vom Tubusverbindungsstutzen 2 am distalen Ende bis zur Spitze 4 am proximalen Ende der Larynxmaske ist diese insgesamt einstückig und entsprechend insgesamt aus demselben Kunststoff gefertigt. In der Ansicht gemäss der Figur 1 erkennt man den umlaufenden Cuff 5 der lediglich am proximalen Ende bei der Spitze 4 durch einen Oesopagealausgang 6 im Durchmesser reduziert ist während am distalen Ende der Cuff 5 unter dem Tubusverbindungsstutzen 2 hindurch läuft. Am proximalen Ende läuft der Oesophagealausgang 6 in einem Art Kanal über den Cuff 5 hinweg.

Der Cuff 5 wird offen gespritzt gefertigt. Insbesondere in den Schnittzeichnungen 6 und 7, aber auch in der Ansicht gemäss der Figur 2 erkennt man eine umlaufende, praktisch dem peripheren Rand des Deckels 3 folgend eine umlaufende, verdickte Klebe-, Schweisswand 13, während gleichzeitig der untere Rand des hier noch offen hergestellt dargestellten Cuffs 5 ebenfalls mit einer umlaufenden Klebe- Schweissrand 15 versehen ist. Erst durch die Verklebung beziehungsweise Verschweissung des Klebe- Schweissrandes 15 mit der entsprechenden umlaufenden Klebe- Schweisswand 13 ergibt sich der geschlossene umlaufende Cuff 5. Dieser Cuff 5 wird über einen Belüftungsstutzen 8, welcher an der ventralen Seite des Tubusverbindungsstutzens 2 ist aufgeblasen oder entlüftet. Dieser Belüftungsstutzens 8 ist in den Figuren 2 - 4 deutlich erkennbar.

Die umlaufende Klebe- beziehungsweise Schweisswand 13 begrenzt unterhalb der Deckplatte 3 einen sogenannten Respirationsraum 7.

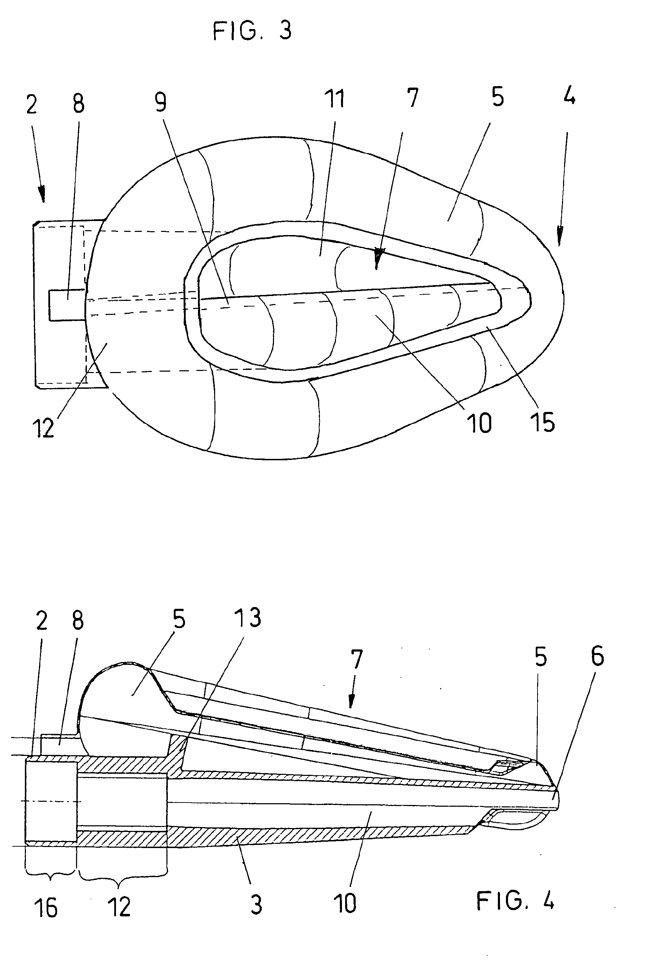

Wie bereits erwähnt wird in den Tubusverbindungsstutzen 2 ein hier nicht dargestellter Einführungstubus eingebracht. Dieser Einführungstubus besteht vorteilhafterweise, jedoch nicht zwingend aus einem einzigen Kunststoffschlauch mit zwei Lumen die entsprechend in der Form und Grösse jenen Lumen angepasst sind die in der Larynxmaske vorhanden sind. Um diese Verbindung der Larynxmaske mit dem Einführungstubus möglichst einfach zu gestalten weist der Tubusverbindungsstutzen 2 einen Steckbereich 16 auf, der insbesondere in den Figuren 2 und 4 erkennbar ist. Der Steckbereich 16 ist nicht in zwei Lumen unterteilt, da bevorzugterweise hier der Einführungstubus eingebracht ist, der diese zwei Lumen aufweist und zwar in der gleichen Gestaltung wie in dem dem Steckbereich 16 nachfolgenden Einführungsstrecke 12. Diese Einführungsstrecke 12 verläuft mindestens annährend unter dem Bereich in dem die beiden Lumen 10 und 11 sich über den Cuff 5 hinweg erstrecken. Zwar erscheint es in der Figur 4 so als ob diese Bereiche unter dem Cuff 5 hindurch verlaufen, doch liegt dies nur an der Darstellung bei der die ventrale Seite statt die dorsale Seite nach oben gerichtet dargestellt ist.

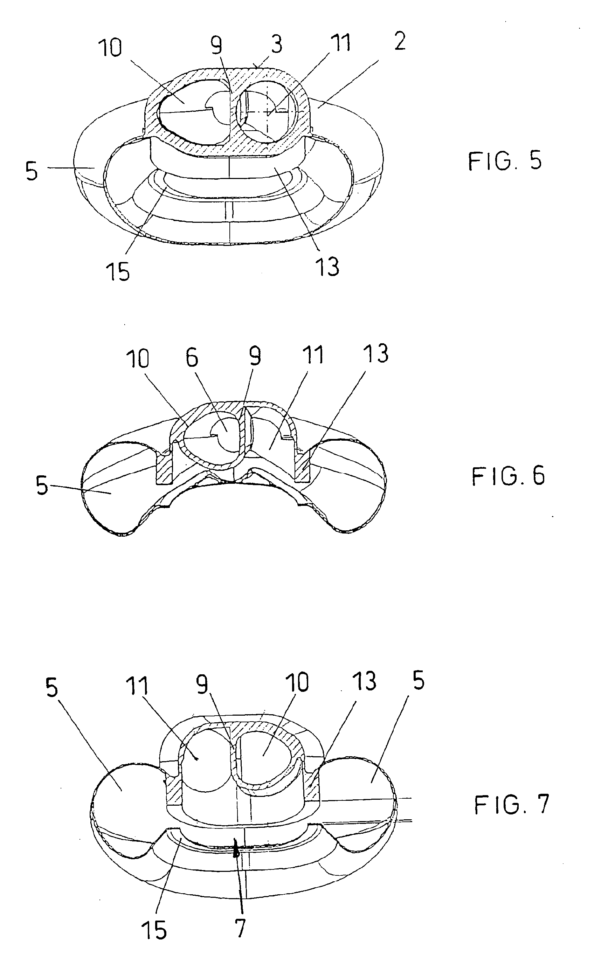

Dem Steckbereich 16 des Tubusverbindungsstutzen 2 folgt wie bereits erwähnt die Einführungsstrecke 12. In diesem Bereich erkennt man zwei getrennte Lumen, die in diesem Bereich beide geschlossen sind, nämlich einerseits den Oesophageallumen 10 und andererseits den Respirationslumen 11. Am deutlichsten ist dies in der Figur 5 ersichtlich. Zwischen diesen beiden Lumen 10 und 11 verläuft mindestens annährend senkrecht zum Deckel 3 eine Trenn- und Stützwand 9. Diese Trenn- und Stützwand 9, welche in etwa in der Mitte längs die Larynxmaske 1 durchsetzt, erhält die Larynxmaske insgesamt eine erhöhte Steifigkeit die einem eventuellen Knicken entgegenwirkt.

Die Trenn- und Stützwand 9 kann in einer vorteilhaften Ausführung einen bis nahe zur Spitze hin verschlossenen sackartigen Zwischenraum aufweisen, welche ein Versteifungselement aufnehmen kann.

Auch die verdickte Klebe-, Schweisswand 13, welche in der Figur 5 nicht geschnitten ist, erhöht die Steifigkeit der Larynxmaske. Man erkennt deutlich, dass der Cuff noch nicht durch Schweissen oder Kleben verschweisst geschlossen ist und entsprechend erkennt man auch die Klebe- beziehungsweise Schweissränder 15.

Nach Beendigung der Einführungsstrecke 12 verläuft der Oesophageallumen 10 auch weiterhin geschlossen, während der Respirationslumen 11 sich zu einem praktisch U-förmigen Kanal öffnet, wie dies in den Figuren 6 und 7 deutlich erkennbar ist. Beide Figuren zeigen denselben Schnitt an derselben Stelle dar, jedoch ist in der Figur 6 die Blickrichtung zum proximalen Ende gerichtet, während in der Figur 7 der Blick zum distalen Ende gerichtet ist. Durch den Wegfall des ventralen Wandbereiches des Respirationslumens 11 verbleibt die Trenn- und Stützwand 9 unverändert. In diesen Figuren erkennt man auch, dass der Durchmesser der beiden Lumen, nämlich des Oesophageallumens 10 und des Respirationslumens 11 und damit auch der Trenn- und Stützwand 9 von der Deckplatte 3 in ventraler Richtung sich weniger weit von der Deckplatte 3 nach unten verläuft als die umlaufende Klebebeziehungsweise Schweisswand 13. Das Ende dieser Klebebeziehungsweise Schweisswand 13 spannt eine Ebene auf und diese Ebene wird weder durch die beiden Lumen 10 und 11 noch durch die Wand 9 durchstossen. Damit verbleibt unterhalb der beiden Lumen 10 und 11 und der Trenn- und Stützwand 9 ein relativ grosser Respirationsraum 7. Man erkennt, dass nach der Einführungsstrecke 12 von wo aus das Respirationslumen 11 sich öffnet, dieser Raum mit dem Respirationsraum 7 direkt ineinander übergeht. In der Figur 6, bei dem der Diametralschnitt durch die Larynxmaske in Blickrichtung zum proximalen Ende gezeigt ist, erkennt man dass das Oesophageallumen 10 zum Oesophagealausgang 6 offen ist, während das Respirationslumen 11 in proximaler Richtung durch die Klebe- beziehungsweise Schweisswand 13 begrenzt wird.

Wie bereits erwähnt ist der Einführungstubus hier nicht dargestellt. Bevorzugterweise wird ein solcher Einführungstubus im Querschnitt etwa die Gestalt haben die der Tubusverbindungsstutzen 2 im Schnittbereich gemäss der Figur 5 aufweist. Dies ist jedoch keineswegs zwingend. Beispielsweise kann auch in den Steckbereich 16 bis zum Absatz 14 ein Adapter eingeschoben werden, in den zwei einzelne Schläuche münden die zusammen einen Einführungstubus 2 bilden.

### Bezugszeichenliste:

- 1: Larynxmaske
- 2: Tubusverbindungsstutzen
- 3: Deckplatte
- 4: Spitze der Larynxmaske
- 5: Cuff
- 6: Oesophagealausgang
- 7: Respirationsraum
- 8: Belüftungsstutzen des Cuff
- 9: Trenn- und Stützwand
- 10: Oesophageallumen
- 11: Respirationslumen
- 12: Einführungsstrecke
- 13: Klebe- Schweisswand
- 14: Absatz
- 15: Klebe- Schweissrand
- 16: Steckbereich

## Patentansprüche

1. Larynxmaske (1) umfassend eine dorsale Deckplatte mit einem daran angeformten umlaufenden, aufblasbaren Cuff (5) und einem Tubusverbindungsstutzen (2) zur Verbindung mit mindestens einem Einführungstubus, welcher ein Luftzufuhrlumen und ein Oesophageallumen umfasst wobei der Tubusverbindungsstutzen (2) eine Einführungsstrecke (12) aufweist, wobei eine in Längsrichtung der Larynxmaske (1) gerade verlaufende, diese vom distalen bis mindestens annähernd zu deren proximalen Ende durchsetzende senkrecht und mindestens annähernd mittig an der Deckplatte angeformte von dorsal nach ventral verlaufende Trenn- und Stützwand (9) aufweist, die die Trennung zwischen einem geschlossenen Lumen als Oesophageallumen (10) und einem sich öffnenden Lumen als Respirationslumen (11) bildet, welches in einem unter der Deckplatte (3) vom Cuff (5) abdichtbaren Respirationsraum (7) mündet ; **dadurch gekennzeichnet, dass** der Respirationsraum (7) durch eine umlaufende Klebe- beziehungsweise Schweisswand (13), die von der Deckplatte (3) zum Cuff (5) hin gerichtet ist, begrenzt ist und die Trenn- und Stützwand (9) sich von der Deckplatte (3) bis maximal in die von der umlaufenden Klebe- beziehungsweise Schweisswand (13) aufgespannten Ebene hinunter erstreckt und wobei ferner die Trenn- und Stützwand (9) am proximalen Ende der Larynxmaske (1) zu einem Oesophagealdurchgang (6) hinläuft, der durch eine Öffnung in der Deckplatte (3) gebildet ist, so dass der Oesophagealdurchgang (6) oberhalb des Cuffs (5) über diesen hinweg sich erstreckt.

2. Larynxmaske (1) nach Anspruch 1, **dadurch gekennzeichnet, dass** der Querschnitt des Respirationslumens (11) grösser ist als der Querschnitt des Oesophageallumen (10) im Bereich der Einführungsstrecke (12).

3. Larynxmaske (1) nach Anspruch 1, **dadurch gekennzeichnet, dass** die Trenn- und Stützwand (9) der Larynxmaske (1) senkrecht zur Deckplatte (3) verläuft.

4. Larynxmaske (1) nach Anspruch 1, **dadurch gekennzeichnet, dass** die Einführungsstrecke (12) durch einen den Querschnitt verengenden Absatz (14) begrenzt ist, der als Anschlag für den einzusteckenden Einführungstubus dient.

5. Larynxmaske (1) nach Anspruch 1, **dadurch gekennzeichnet, dass** die Trenn- und Stützwand (9) einen nach proximal abgeschlossenen sackartigen Zwischenraum für die Aufnahme eines Versteifungselementes verfügt.

## Claims

1. Larynx mask (1) comprising a dorsal cover plate with a circumferential inflatable cuff (5) formed thereon and a tube connecting connector (2) for connecting to at least one insertion tube, which comprises an air supply lumen and an oesophageal lumen, whereby the tube connecting connector (2) has an insertion section (12), wherein there is a separating and supporting wall (9) running from dorsal to ventral, which is formed at least approximately centrally on the cover plate and runs in the longitudinal direction of the larynx mask (1) passing through it from distal to at least approximately its dorsal end and forming the partition between a closed lumen as the oesophageal lumen (10) and an open lumen as a respiration lumen (11), which opens out into a respiration chamber (7) which can be sealed under the cover plate (3) by the cuff (5), **characterised in that** the respiration chamber (7) is delimited by a circumferential adhesive or welding wall (13) which runs from the cover plate (3) to the cuff (5), and the separating and supporting wall (9) extends from the cover plate (3) to maximally down into the plane spanned by the circumferential adhesive or welding wall (13) and whereby at the proximal end of the larynx mask (1) the separating and supporting wall (9) runs to an oesophageal outlet (6) which is formed by an opening in the cover plate (3) so that the oesophageal outlet (6) extends above it over the cuff (5).

2. Larynx mask (1) in accordance with claim 1 **characterised in that** the cross-section of the respiration lumen (11) is larger than the cross-section of the oesophageal lumen (10) in the opening area of the insertion section (12).

3. Larynx mask (1) in accordance with claim 1 **characterised in that** the separating and supporting wall (9) of the larynx mask (1) runs perpendicularly to the cover plate (3).

4. Larynx mask (1) in accordance with claim 1 **characterised in that** the insertion section (12) is limited by a projection (14) narrowing the cross-section and serving as a stop for the insertion tube to be inserted.

5. Larynx mask (1) in accordance with claim 1 **characterised in that** the separating and supporting wall (9) has a proximally closed, sack-like intermediate space for accommodating a reinforcing element.

## Revendications

1. Masque laryngé (1) comprenant une plaque supérieure dorsale avec un coussinet (5) gonflable circulaire formé dessus et une tubulure de liaison de tube (2) pour la liaison à au moins un tube d'introduction qui comprend un passage d'amenée d'air et un passage oesophagien, sachant que la tubulure de liaison de tube (2) présente une voie d'introduction (12), sachant qu'elle présente dans le sens longitudinal du masque laryngé (1) une paroi de séparation et d'appui (9) dorsale à ventrale droite, traversant celui-ci de l'extrémité distale à au moins presque son extrémité proximale et formée au moins presque au centre sur la plaque supérieure, qui forme la séparation entre un passage fermé en tant que passage d'oesophage (10) et un passage s'ouvrant en tant que passage respiratoire (11), qui débouche dans un espace respiratoire sous la plaque supérieure (3) pouvant être étanche par rapport au coussinet (5), **caractérisé en ce que** l'espace respiratoire (7) est délimité par une paroi collée, respectivement soudée, périphérique (13) qui est dirigée de la plaque supérieure (3) vers le coussinet (5), et la paroi de séparation et d'appui (9) s'étend par le dessous, de la plaque supérieure (3) jusqu'à maximum dans le plan fixé par la paroi collée, respectivement soudée, périphérique (13), et sachant qu'en outre la paroi de séparation et d'appui (9) part sur l'extrémité proximale du masque laryngé (1), vers une percée oesophagienne (6) qui est formée par une ouverture dans la plaque supérieure (3), de telle sorte que la percée oesophagienne (6) s'étend au-dessus du coussinet (5) au-delà de celui-ci.

2. Masque laryngé (1) selon la revendication 1, **caractérisé en ce que** la section transversale du passage respiratoire (11) est supérieure à la section transversale du passage oesophagien (10) au niveau de la voie d'introduction (12).

3. Masque laryngé (1) selon la revendication 1, **caractérisé en ce que** la paroi de séparation et d'appui (9) du masque laryngé (1) est verticale à la plaque supérieure (3).

4. Masque laryngé (1) selon la revendication 1, **caractérisé en ce que** la voie d'introduction (12) est délimitée par un épaulement (14) réduisant la section transversale qui sert d'épaulement au tube d'introduction à insérer.

5. Masque laryngé (1) selon la revendication 1, **caractérisé en ce que** la paroi de séparation et d'appui (9) possède un espace intermédiaire en forme de poche fermé dans le sens proximal, pour recevoir un élément de renfort.
